# EUROPÄISCHE PATENTSCHRIFT

(11) **EP 2 018 870 B1**
(45) Veröffentlichungstag und Bekanntmachung des Hinweises auf die Patenterteilung: **19.10.2011**
(21) Anmeldenummer: 08450107.1
(22) Anmeldetag: 15.07.2008
(51) Int. Cl.: A61K 36/77

(54) **Verfahren zum Gewinnen von Flavonoiden aus Rosskastanien**
Method for extracting flavonoids from horse chestnuts
Procédé de gain de flavonoïdes à partir de marrons d'Inde

(30) Priorität: 16.07.2007 AT 11152007
(43) Veröffentlichungstag der Anmeldung: 28.01.2009
(73) Patentinhaber: Med'Arom Prof. Dr. Franz OG, 1190 Wien (AT)
(72) Erfinder: Oleszek, Wieslav, 24100 Pulawy (PL)
(74) Vertreter: Haffner und Keschmann Patentanwälte OG

(56) Entgegenhaltungen:
- WO-A1-2006/030567
- DE-A1- 10 112 168
- WOLLINA UWE ET AL: "A review of the microcirculation in skin in patients with chronic venous insufficiency: the problem and the evidence available for therapeutic options" INTERNATIONAL JOURNAL OF LOWER EXTREMITY WOUNDS, SAGE SCIENCE PRESS, THOUSAND OAKS, CA, US, Bd. 5, Nr. 3, 1. September 2006 (2006-09-01), Seiten 169-180, XP009107746 ISSN: 1534-7346

## Beschreibung

Die Erfindung bezieht sich auf ein Verfahren zum Gewinnen von Flavonoiden aus Rosskastanien, wobei in einer ersten Extraktionsstufe Glykoside, essentielle Öle, Cumarinderivate und Tannine extrahiert werden, die extrahierte Fraktion getrocknet und mit Wasser gewaschen wird.

Rosskastanienextrakte finden in der pharmazeutischen und kosmetischen Industrie breite Verwendung, wobei bisher vor allem die Saponine, und unter diesen vor allem Aescin extrahiert wurden. Untersuchungen der Bestandteile von Rosskastanien haben unterschiedliche Werte ergeben. Bekannt ist allerdings, dass Rosskastanien neben den in der pharmazeutischen bzw. kosmetischen Industrie benötigten Saponinen, essentiellen Ölen und neben Cumarinderivaten und Tanninen auch weitere Wertstoffe enthalten, deren Extraktion bisher nicht vorgenommen wurde, da ihre Konzentration als relativ gering angenommen wurde. Bei den bisher bekannten Extraktionsverfahren wurden daher Rosskastanien und insbesondere Schalen von Rosskastanien mit Ethanol extrahiert und die erhaltenen Extrakte nach Abdampfen des Lösungsmittels unter Ausbildung eines Rohextraktpulvers gewaschen. Das erhaltene Waschwasser wurde verworfen.

Es wäre überaus erstrebenswert, die nach der Extraktion von Wertstoffen für die kosmetische und pharmazeutische Industrie verbleibenden Substanzen zu gewinnen und auf diese Weise ein Abfallprodukt, nämlich das nach der ethanolischen Extraktion erhaltenen Abwasser noch sinnbringend zu nutzen. Aus mehreren Literaturstellen war es nämlich bekannt, dass Rosskastanienextrakte auch eine Reihe von Wertstoffen wie Flavonoide, und insbesondere Glykoside von Quercetin und Kaempferol enthalten, wobei diesen Stoffen u.a. eine gute Wirkung als Sauerstofffänger bzw. als Radikalfänger zugeschrieben wurde. Flavonoide werden in der Literatur auch als Bakterizide, Viruzide und Antioxidantien beschrieben und insbesondere bei der Behandlung von Varikose und anderen Anomalien der Gefäße in der pharmazeutischen Industrie bereits eingesetzt.

Die Erfindung zielt nun darauf ab, derartige Wirkstoffe, die bisher aus Rosskastanien nicht gewonnen wurden, aus einem Abfallprodukt, nämlich dem Waschwasser der zuvor beschriebenen, üblicherweise durchgeführten Extraktion zu gewinnen und einer wirtschaftlichen Verwendung sowie einer entsprechenden Reinigung zuzuführen. Zur Lösung dieser Aufgabe besteht das erfindungsgemäße Verfahren im Wesentlichen darin, dass ausgehend von dem zuvor beschriebenen Waschwasser die Waschwasserfraktion eingeengt und getrocknet wird und aus dem Rückstand der Waschwasserfraktion die Flavonoide gewonnen werden. Dadurch, dass einem bekannten Extraktionsverfahren nachgeschaltet, der Rückstand des Waschwassers einer nochmaligen Extraktion unterworfen wird, gelingt es, Flavonoide in nennenswerten Mengen zu gewinnen, sodass sich ein solches Verfahren zur Gewinnung von Flavonoiden wirtschaftlich durchführen lässt.

Mit Vorteil wird das erfindungsgemäße Verfahren so durchgeführt, dass die Trocknung der wässrigen Phase durch Abdampfen unter reduziertem Druck bei Temperaturen unter 60° C, insbesondere etwa 50° C vorgenommen wird, wodurch die thermische Belastung der Wirkstoffe verringert wird.

Das erfindungsgemäße Verfahren wird vorteilhaft so durchgeführt, dass die Reinigung des Rückstands der Waschwasserfraktion unter Verwendung von Chromatographie-Säulen, wie beispielsweise Glassäulen enthaltend irreguläres Kieselgel, vorgenommen wird, wobei in einem ersten Schritt von Flavonoiden verschiedene polare Verbindungen mit Wasser ausgewaschen werden und anschließend Flavonoide mit Alkohol, insbesondere Methanol, eluiert werden. Derartige Chromatographie-Säulen enthaltend irreguläres Kieselgel werden beispielsweise unter dem Markennamen LiChroprep RP-18 vertrieben. Das Reinigungsverfahren nützt hierbei den Umstand, dass in der wässrigen Phase eine Reihe von polaren Verbindungen enthalten ist, deren Polarität sich wesentlich voneinander unterscheidet. In einem ersten Schritt, in welchem die Säule mit Wasser gewaschen wird, werden somit diejenigen polaren Verbindungen eluiert, welche in wässrigem Medium nicht von der Säule zurückgehalten werden können. Die Elution der anderen Verbindungen mit Alkoholen und insbesondere mit 40 %-igem Methylalkohol ergibt nun überraschenderweise hochkonzentrierte Fraktionen der gewünschten Flavonoide, wodurch auf diese Weise eine selektive, präparative Methode bereitgestellt wird, aus den bisher als Abfall gehandhabten Waschwässern Wertstoffe zu gewinnen.

Mit Vorteil werden die gereinigten Flavonoide auf chromatographischem Weg unter Verwendung eines MeOH-Gradienten getrennt.

Das erfindungsgemäße Verfahren wird nachfolgend anhand eines Ausführungsbeispiels näher erläutert.

Zur Herstellung des erfindungsgemäß einzusetzenden Ausgangsmaterials wurde nicht nur auf Waschwässer aus einschlägigen Extraktionsbetrieben zurückgegriffen, sondern ein diesen Abwässern entsprechendes Abwasser wie folgt hergestellt:
A. hippocastanum-Samen, d.h. Rosskastanien wurden zerstoßen und zu Pulver verrieben, wobei 250 g dieser Substanz mit 80 %-igem Methanol bei Raumtemperatur extrahiert wurden. Nach 48h wurde der Extrakt gefiltert und die Rückstände wurden nochmals über den Zeitraum von 24h extrahiert. Die Extrakte wurden vereinigt und das Lösungsmittel unter reduziertem Druck abgetrennt, wodurch ein Rohextrakt in der Menge von 63 g gebildet wurde.

Von diesem Rohextrakt wurden in der Folge 10 g in Wasser suspendiert. Dieser Schritt entspricht dem Waschschritt, wie er zuvor bei der Gewinnung der bisher extrahierten essentiellen Öle, Saponine und/oder dgl. verwendet wurde. Die Suspension führt zu einem wässrigen Eluat, welches dem beschriebenen Waschwasser entspricht. Dieses Waschwasser wurde nun auf eine 6 cm x 10 cm 40-64 µm LiChroprep RP-18 Glassäule aufgegeben, welche zuvor mit Wasser konditioniert wurde. In einem ersten Waschschritt wurde Wasser eingesetzt, und der Großteil polarer Bestandteile ausgewaschen. In der Folge wurde mit 40 %-igem Methanol eluiert, wobei ein Flavonoid-Eluat gebildet wurde. Dieses Eluat wurde in der Folge eingedampft, wobei unter reduziertem Druck bei 50° C rohes Flavonoidpulver in einer Menge von 3,67 g gebildet wurde. In analoger Weise wurde auch das von Extraktionsbetrieben zur Verfügung gestellte Abwasser behandelt, um die Flavonoide zu gewinnen.

In der Folge wurde das Flavonoidpulver nochmals in destilliertem Wasser suspendiert und einer 3 cm x 40 cm 40-64 µm LiChroprep RP-18 Glassäule aufgeben (Millipore Corp., Bedford, MA). Die Kolonne wurde mit einem 0-100 %-igen, linearen Gradienten von Methanol entwickelt, wobei 10 ml- Fraktionen gesammelt, mit Dünnschichtchromatographie getrennt, in 15 %-iger Essigsäure entwickelt und unter UV-Licht bei einer Wellenlänge von 366 nm observiert wurden. Fraktionen, die die gleichen Dünnschichtchromatographiemuster auswiesen (55 Fraktionen) wurden in der Folge weiter analysiert, wobei eine Ultraperformance Liquid Chromatographie (UPLC) Analyse durchgeführt wurde. Es wurden unterschiedliche Substanzen identifiziert, welche zumeist als gelbe amorphe Pulver gewonnen wurden. Fraktionen, welche mehr als ein Produkt enthielten, wurden weiter auf einer RP-18 Glassäule (2 cm x 50 cm 40-63 µm) mit einem isokratischen System (AcN-1 % H₃PO₄) aufgetrennt. Die einzelnen individuellen Flavonoide und deren Strukturen wurden in der Folge durch Massenspektrometrie und H₁- sowie C₁₃-NMR identifiziert. Im Einzelnen wurden hier mehrere Verbindungen differenziert, deren spektrale Eigenschaften in Tabelle 1 wiedergegeben sind.

**Tabelle 1**

| UPLC-Daten der analysierten Flavonoide und ihre ESI/MS/MS Fragmentierungsprofile | | | |
|---|---|---|---|
| Verbindung | Rückhaltezeit (Min) | UV (nm) | ESI/MS/MS |
| 1 | 1.15 | 266,344 | 919 [M-H]⁻, 757 [M-Glc-H]⁻, 625 [M-Glc-Xy1]⁻, 595 [M-2G1c-H]⁻, 463 [M-2Glc-Xyl-H]⁻, 300 [quercetin-2H)⁻ |
| 2 | 1.26 | 265,349 | 757 [M-H]⁻, 625 [M-Xyl-H]⁻, 595 [M-G1c-H]⁻, 463 [M-Glc-Xy]⁻, 300 [quercetin-2H]⁻ |
| 3 | 1.75 | 255,353 | 757 [M-H]⁻, 595 [M-Glc]⁻, 463 [M-Glc-Xyl-H]⁻, 301 [quercetin-H]⁻ |
| 4 | 1.91 | 255,353 | 595 [M-H]⁻, 463 [M-Xyl-H]⁻, 301 [quercetin-H]⁻ |
| 5 | 2.27 | 265,355 | 863 [M-H]⁻, 595 [M-Glc-105-H]⁻, 300 [quercetin-2H]⁻ |
| 6 | 2.48 | 265,355 | 946 [M-H]⁻, 595 [M-Glc-189-H]⁻, 300 [quercetin-2H]⁻ |
| 7 | 2.54 | 265,346 | 741 [M-H]⁻, 609 [M-Xyl-H]⁻, 447 [M-Xyl-Glc-H]⁻, 285 [kaempferol-H]⁻ |
| 8 | 2.68 | 265,346 | 579 [M-H]⁻, 447 [M-Xyl-H]⁻, 285 [kaempferol-H]⁻ |
| 9 | 2.78 | 265,355 | 771 [M-H]⁻, 639 [M-Xyl-H]⁻, 609 [M-Glc-H]⁻, 314 [M-Xyl-2Glc-H]⁻, 300 [quercetin-2H]⁻ |
| 10 | 2.95 | 266,351 | 771 [M-B]⁻, 625 [M-Rha-H]⁻, 609 [M-Glc-H]⁻, 463 [M-Glc-Rha-H]⁻, 300 [quercetin-2H]⁻ |
| 11 | 3.32 | 255,344 | 609 [M-H]⁻, 447 [M-Glc-H]⁻, [quercetin-H]⁻ |
| 12 | 3.65 | 266,344 | 930 [M-H]⁻, 595 [M-335-H]⁻, 300 [quercetin-2H]⁻ |
| 13 | 4.14 | 264,344 | 593 [M-H]⁻, 431 [M-G1c-H]⁻, 285 [kaempferol-H]⁻ |

Für die UPLC-Analyse kam der Acquinity Ultra Performance Liquid Chromatograph (Waters) zum Einsatz. Das Profil wurde auf einer UPLC BEH C₁₈-Säule (1,7 µm, 50 mm x 2,1 mm) unter Anwendung eines gradiellen Induktionsprofils und einer mobilen Phase, bestehend aus 0,1 % Essigsäure in Wasser und 40 % AcN erstellt. Die Säule wurde bei 50°C gehalten und die Flussrate wurde bei 0,35 ml/Min konstant gehalten. Ein Gramm getrockneter und fein zerstoßener Rosskastanien wurde über Nacht mit 50 ml 80% Ethanol bei Raumtemperatur extrahiert. Der Extrakt wurde gefiltert und der Rückstand zusätzlich 2 Mal mit 50 ml 80% Ethanol unter Rückflussbedingungen für eine Stunde extrahiert. Die Extrakte wurden kombiniert und das Lösungsmittel unter reduziertem Druck entfernt. Der Rohextrakt wurde in Wasser (10 ml) suspendiert und 2 ml wurden durch eine C₁₈Sep-Pack-Patrone (Waters Associates), welche mit Wasser konditioniert war, geleitet. Die Patrone wurde zuerst mit Wasser gereinigt, um Zucker zu entfernen, worauf die Elution mit 40%-igem Methanol erfolgte. Diese Fraktion wurde evaporiert und in 1 ml Ethanol für die Analysen gelöst. Die Bestimmung der individuellen Verbindung in dem Profil wurde durch Versetzen des Extrakts mit gereinigtem Standard durchgeführt. Die Berechnungen wurden auf Basis externer Standardkurven, welche für die einzelnen Verbindungen aufgestellt wurden, durchgeführt.

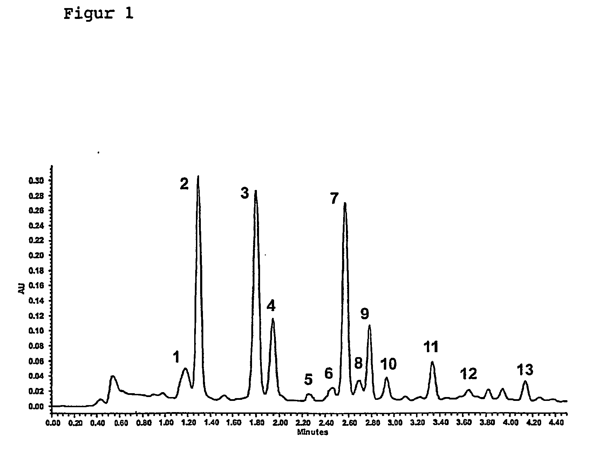

Die Extraktion von Rosskastanien mit wässrigem Ethanol gefolgt von einer Niederdrucksäulenchromatographie lieferte mehrere Flavonoide. Ihre Strukturen wurden mittels Massenspektrometrie und ¹H- und ¹³C-NMR aufgeklärt. Auf diese Weise wurden sieben Flavonoide identifiziert, welche sechs bekannte Verbindungen (2, 3, 4, 7, 11 und 13 in Figur 1) umfassten, welche schon zuvor in *A. hippocastanum* und *A. chinensis* aufgefunden worden waren. Ihre MS- und NMR-Daten waren mit den beschriebenen identisch. Die Verbindung 9 wurde als Tamarixetin 3-O-[β-D-glucopyranosyl(1→3)]-*O-*β-D-xylopyranosyl-(1→2)-*O*-β-D-glucopyranosid identifiziert und stellt nach unserem Dafürhalten eine neue bisher noch nicht in anderen Pflanzen identifizierte Verbindung dar.

Die isolierten Verbindungen wurden für die Erstellung eines chromatographischen Profils der Flavonoide aus Rosskastanien verwendet. UPLC erwies sich für die Trennungen aller Verbindungen als geeignet, wobei die Trennung in einem sehr kurzen Lauf, welcher nicht länger als 4,5 Min dauerte, durchgeführt. Es stellte sich heraus, dass alle isolierten Verbindungen dominante Flavonoide des Rosskastanienextrakts waren. Das Elutionsprofil der Flavonoide ist in Fig. 1 dargestellt, welches den Daten in Tabelle 1 entspricht.

Das UPLC-Profil der Rosskastanienflavonoide zeigt, dass Di- und Triglycoside von Quercetin und Kämpferol die Hauptkomponenten sind und in Spuren acyliert vorkommen. Die Gesamtkonzentration der Flavonoide in Rosskastanien lag bei 0,88% der Trockenmasse, was im Widerspruch zu den bisherigen Daten steht, welche lediglich ca. 0,3% (berechnet als Rutin) als Gesamtkonzentration anführten. Somit zeigt sich, dass bei einer spezialisierten Extraktion die tatsächliche Konzentration der Flavonoide in Rosskastanien mehr als zwei Mal höher als bisher angenommen liegt. Der alkoholische Extrakt enthielt bis zu 3,5% Flavonoide bezogen auf die Trockenmasse, was eine bedeutende Konzentration darstellt, die die Gesamtaktivität des Extraktes beeinflussen könnte. Diese Konzentration konnte auf bis zu knapp 10% der Trockenmasse gesteigert werden, wenn der Extrakt auf einer C18-RP-Säule gereinigt wurde. Das Flavonoidprofil der Waschwasserfraktion war mit dem Profil des Rohextraktes identisch, wobei die Gesamtkonzentration bei 2,54% der Trockenmasse lag. Die Einschrittreinigung dieser Fraktion lieferte die Rosskastanienflavonoide der Waschwasserfraktion mit einer Flavonoidkonzentration von 11,23% der Trockenmasse. Dies ist ein ziemlich hoher Wert und das Produkt kann somit einer kommerziellen Verwertung in der pharmazeutischen, kosmetischen und der Lebensmittelindustrie zugeführt werden.

## Patentansprüche

1. Verfahren zum Gewinnen von Flavonoiden aus Rosskastanien, wobei nach einer ersten Extraktionsstufe Glykoside, essentielle Öle, Cumarinderivate und Tannine extrahiert werden, die extrahierte Fraktion getrocknet und mit Wasser gewaschen wird, **dadurch gekennzeichnet, dass** die Waschwasserfraktion eingeengt und getrocknet wird und aus dem Rückstand der Waschwasserfraktion die Flavonoide gewonnen werden.

2. Verfahren nach Anspruch 1, **dadurch gekennzeichnet, dass** die Trocknung der wässrigen Phase durch Abdampfen unter reduziertem Druck bei Temperaturen unter 60° C, insbesondere etwa 50° C vorgenommen wird.

3. Verfahren nach Anspruch 1 oder 2, **dadurch gekennzeichnet, dass** die Reinigung des Rückstands der Waschwasserfraktion unter Verwendung von Chromatographie-Säulen, wie beispielsweise Glassäulen enthaltend irreguläres Kieselgel, vorgenommen wird, wobei in einem ersten Schritt von Flavonoiden verschiedene polare Verbindungen mit Wasser ausgewaschen werden und anschließend Flavonoide mit Alkohol, insbesondere Methanol, eluiert werden.

4. Verfahren nach Anspruch 3, **dadurch gekennzeichnet, dass** die gereinigten Flavonoide auf chromatographischem Weg unter Verwendung eines Methanol-Gradienten getrennt werden.

## Claims

1. Method for recovering flavonoids from chestnut, in which after a first extraction-stage glycosides, essential oils, derivatives of cumarin and tannins are extracted and the extracted fraction is dried and washed with water, **characterized in that** the washing water-fraction is concentrated and dried and the flavonoids are recovered from the remainder of the washing water-fraction.

2. Method according to claim 1, **characterized in that** drying of the aqueous phase is carried out by vaporisation at reduced pressure at temperatures below 60°C, in particular at about 50°C.

3. Method according to claim 1 or 2, characterized that the purification of the remainder of the washing water-fraction is carried out by using chromatographic columns, as for example glass-columns containing irregular silica gel, wherein in a first step polar compounds different from flavonoids are washed out with water and flavonoids are subsequently eluted with alcohol, in particular methanol.

4. Method according to claim 3, **characterized in that** the purified flavonoids are chromatographically separated by using a methanol gradient.

## Revendications

1. Procédé pour récupérer des flavonoïdes a partir de marrons, dans lequel suivant une première étape d'extraction des glycosides, des huiles essentielles, des dérivées de cumarin et des tannins son extrait, la fraction extraite est séchée et nettoyée avec de l'eau, **caractérisée en ce que** la fraction de l'eau de nettoyage est réduite et séchée et les flavonoïdes sont récupérées à partir du résidu de la fraction de l'eau de nettoyage.

2. Procédé selon la revendication 1, **caractérisée en ce que** le séchage de la phase aqueuse est effectué par évaporation sous pression réduite á des températures moins de 60°C, en particulier environ 50°C.

3. Procédé selon la revendication 1 ou 2, **caractérisée en ce que** le nettoyage du résidu de la fraction de l'eau de nettoyage est effectué en utilisant des colonnes de chromatographie, par exemple des colonnes en verre contenant de gel de silice irrégulier, en ravinant dans une première étape des composés polaires différent aux flavonoïdes et des flavonoïdes sont ensuite ravinés avec de l'alcool, en particulier du méthanol.

4. Procédé selon la revendication 3, **caractérisée en ce que** les flavonoïdes nettoyés sont séparés par chromatographie en utilisant en gradient de méthanol.
